Europäisches Patentamt

European Patent Office

Office européen des brevets

(19)

(11) Veröffentlichungsnummer: **0 055 357**
**B1**

(12) **EUROPÄISCHE PATENTSCHRIFT**

(45) Veröffentlichungstag der Patentschrift:
11.01.84

(21) Anmeldenummer: 81108843.4

(22) Anmeldetag: 24.10.81

(51) Int. Cl.³: **C 07 C 59/70,** C 07 C 59/68,
C 07 C 51/363, C 07 C 51/367

(54) Verfahren zur Herstellung von chlorierten Phenoxyalkansäuren.

(30) Priorität: 31.12.80 DE 3049541

(43) Veröffentlichungstag der Anmeldung:
07.07.82 Patentblatt 82/27

(45) Bekanntmachung des Hinweises auf die Patenterteilung:
11.01.84 Patentblatt 84/2

(84) Benannte Vertragsstaaten:
AT BE CH DE FR GB IT LI LU NL SE

(56) Entgegenhaltungen:
DE - B - 1 027 680
FR - A - 1 116 266
GB - A - 688 659
GB - A - 855 504

SOVIET INVENTIONS ILLUSTRATED, Derwent
Publications, Section I, Chemical, März 1969,
Zusammenfassung 220978, Teil 5 LONDON (GB) A.L.
ENGLIN et al.: "2,4-Dichlorophenoxyacetic acid",
Seiten 6 und 7

(73) Patentinhaber: BASF Aktiengesellschaft,
Carl-Bosch-Strasse 38, D-6700 Ludwigshafen (DE)

(72) Erfinder: Husslein, Gerd, Dr., Herrenbergstrasse 2,
D-6702 Bad Duerkheim (DE)
Erfinder: Hamprecht, Gerhard, Dr.,
Rote-Turm-Strasse 28, D-6940 Weinheim (DE)
Erfinder: Koenig, Karl-Heinz, Dr., Pierstrasse 8 A,
D-6710 Frankenthal (DE)
Erfinder: Boehm, Walter, Dr., Mittlerer Waldweg 11,
D-6719 Kirchheim (DE)
Erfinder: Gaeng, Manfred, Dr., An der Tuchbleiche 11,
D-6712 Bobenheim-Roxheim (DE)

### Verfahren zur Herstellung von chlorierten Phenoxyalkansäuren

Die vorliegende Erfindung betrifft ein verbessertes Verfahren zur Herstellung von 2,4-Dichlor- bzw. 2-Methyl-4-chlorphenoxyessigsäure oder alpha-2,4-Dichlor- bzw. alpha-2-Methyl-4-chlorphenoxypropionsäure durch Umsetzung einer wäßrigen Suspension von Phenoxyessigsäure bzw. 2-Methylphenoxyessigsäure oder alpha-Phenoxypropionsäure bzw. alpha-2-Methylphenoxypropionsäure mit Chlorgas.

Aus Zhurnal Prikladnoi Khimii, Vol. 43 (1970), No. 12, Seiten 2686 – 2692 (engl. Übersetzung ibd. Seiten 2726 – 2730) ist es bekannt, bei der Chlorierung von Phenoxyessigsäure mit Chlorgas im Labormaßstab bei 75° C in Wasser nach 4- bis 6stündiger Reaktionszeit eine Ausbeute von maximal 96% der Theorie an 2,4-Dichlorphenoxyessigsäure (gaschromatographisch als Ester bestimmt) zu erhalten. Für die industrielle Produktion dieses Stoffes nach dem geschilderten Verfahren ergeben sich folgende Nachteile:

1. Die Verwendung verflüssigten Chlorgases.
2. Bei der Reaktionstemperatur bilden sich schwer rührbare Schmelzen von Phenoxyessigsäure in Wasser, die nur schlecht chloriert werden können.
3. Die Raum-Zeit-Ausbeute ist zu niedrig, weil die Chlorierung zu langsam und in zu großer Verdünnung erfolgt.
4. Nebenreaktionen senken die Produktqualität. Beispielsweise besitzt das Produkt infolge saurer Hydrolyse von Ausgangs- und Endstoff den unangenehmen Geruch von 2,4-Dichlorphenol.
5. Chlorverluste bei der Umsetzung, d. h., freies Chlorgas im Abgas, komplizieren die Reinigung des Abgases.

Es ist aus der russischen Patentschrift 220 978 bekannt, bei der Umsetzung eine Chlorierungstemperatur von 60 – 65° C einzuhalten, das Ausgangsprodukt in Form einer feinen Suspension (Teilchendurchmesser 0,5 mm) anzuwenden und in Gegenwart von viel Wasser (ca. 1 mol Phenoxyessigsäure je 300 mol Wasser) zu arbeiten. Dieses Verfahren liefert das Endprodukt mit einer Reinheit von maximal 80%, was für die Praxis völlig unzureichend ist.

Es ist ferner bekannt, die Chlorierung mit Hilfe von Alkalihypochlorit in saurem Milieu in heterogener Phase durchzuführen, wobei das Chlor in situ im Reaktionsmedium durch die Umsetzung von Alkalihypochlorit mit Salzsäure erzeugt wird (DE-B-1 027 680).

Es ist bekannt, die Chlorierung mit Chlorgas in wäßriger Phase durchzuführen, wobei die Säure in Form der wäßrigen Lösung ihres Salzes vorliegt und während der Chlorierung Alkali zu der Lösung zugesetzt wird (FR-A-1 116 266, GB-A-688 659).

Es ist bekannt, die Chlorierung mit Chlorgas in wäßriger Phase durchzuführen, wobei die Säure in Form der wäßrigen Lösung ihres Salzes vorliegt und die Chlorierung in Gegenwart von Natriumbicarbonat durchgeführt wird (GB-A-855 504).

Es wurde nun gefunden, daß man 2,4-Dichlor- bzw. 2-Methyl-4-chlorphenoxyessigsäure oder alpha-2,4-Dichlor- bzw. alpha-2-Methyl-4-chlorphenoxypropionsäure in einer Ausbeute von mindestens 96% d. Th. und einer Reinheit von mindestens 97% erhält, wenn man Phenoxyessigsäure bzw. 2-Methylphenoxyessigsäure oder alpha-Phenoxypropionsäure bzw. alpha-2-Methylphenoxypropionsäure aus ihrer wäßrigen Lösung ausfällt, in die so erhaltene Suspension von Phenoxyessigsäure bzw. 2-Methylphenoxyessigsäure oder alpha-Phenoxypropionsäure bzw. alpha-2-Methylphenoxypropionsäure in Wasser Chlorgas bei einer Temperatur von 30 bis 70° C höchstens in der Menge und Geschwindigkeit, in der es von der Suspension verbraucht wird, einleitet, bis 100 bis 110% der theoretisch erforderlichen Menge an Chlorgas von der Suspension aufgenommen worden sind, und die 2,4-Dichlor- bzw. 2-Methyl-4-chlorphenoxyessigsäure oder alpha-2,4-Dichlor- bzw. alpha-2-Methyl-4-chlorphenoxypropionsäure von der Suspension abtrennt.

Die Ausfällung der Phenoxyessigsäure bzw. 2-Methylphenoxyessigsäure oder alpha-Phenoxypropionsäure bzw. alpha-2-Methylphenoxypropionsäure aus ihrer Lösung kann beispielsweise durch Säurezusatz oder durch Einleiten von Chlorgas erfolgen. Säurezusatz ist beispielsweise der Zusatz von Salzsäure zu der wäßrigen Lösung, bis eine Ausfällung erfolgt. Die Einleitung des Chlorgases erfolgt höchstens mit der Geschwindigkeit, mit der es von der Suspension aufgenommen wird und für die Umsetzung verbraucht wird. Vorteilhaft läßt man das Chlorgas möglichst lange mit der Suspension in Berührung kommen, indem diese beispielsweise stark gerührt wird oder das Chlorgas eine dicke Schicht der Suspension durchdringen muß.

Unter der Lösung der Phenoxyessigsäure bzw. der 2-Methylphenoxyessigsäure oder alpha-Phenoxypropionsäure bzw. alpha-2-Methylphenoxypropionsäure in Wasser ist die Lösung sowohl der Säuren selbst als auch die Lösung ihrer Alkalisalze, z. B. der Natriumsalze oder Kaliumsalze oder ihrer Mischung, zu verstehen.

Die Abtrennung des Endproduktes von der Reaktionsmischung kann beispielsweise durch Abfiltrieren erfolgen.

Für die Umsetzung kann vorteilhaft eine wäßrige Lösung von Phenoxyessigsäure bzw. 2-Methylphenoxyessigsäure oder alpha-Phenoxypropionsäure bzw. alpha-2-Methylphenoxypropionsäure verwendet werden, die durch Umsetzung von Phenol oder 2-Methylphenol bzw. ihren Alkalisalzen mit Chloressigsäure oder alpha-Chlorpropionsäure in einem organischen Lösungsmittel und Extraktion der so erhaltenen Phenoxyessigsäure bzw. 2-Methylphenoxyessigsäure oder alpha-Phenoxypropionsäure bzw. alpha-2-Methylphenoxypropionsäure aus dem organischen Lösungsmittel mit Wasser erhalten wurde. Hierdurch werden erhebliche Verluste z. B. an Phenoxyessigsäure vermieden, die bei den bekannten Verfahren durch die Verwendung einer festen Phenoxyessigsäure entstehen, die aus der bei ihrer Herstellung entstandenen Lösung abgetrennt worden ist.

Es ist überraschend, daß beim erfindungsgemäßen Verfahren bei den niedrigen Temperaturen und hohen Einsatzstoffkonzentrationen (ca. 1 mol Phenoxyessigsäure oder alpha-Phenoxypropionsäure in 20 – 40 mol Wasser) eine große Reaktionsgeschwindigkeit erzielt wird, die zu einem Produkt hoher Reinheit (mehr als 97%) führt. Es ist also nicht notwendig, eine feine Suspension des Ausgangsproduktes bestimmter Korngröße, wie in der russischen Patentschrift Nr. 220 978 beschrieben, beispielsweise durch Mahlung des festen Ausgangsproduktes herzustellen. Obwohl also keine Mahlung vorgenommen wird, kann die Konzentration des Einsatzstoffes beim erfindungsgemäßen Verfahren gegenüber dem bekannten Verfahren auf das 10fache gesteigert werden.

Durch die Anpassung der Chlorgaszugabe an die Reaktionsgeschwindigkeit sowie durch Ausfällung der Phenoxyessigsäure bzw. der 2-Methylphenoxyessigsäure oder alpha-Phenoxypropionsäure bzw. alpha-2-Methylphenoxypropionsäure aus ihrer wäßrigen Lösung wird beim erfindungsgemäßen Verfahren die Reaktionszeit gegenüber den bekannten Verfahren um mehr als die Hälfte verkürzt. Dies wird unterstützt durch eine Erhöhung der Verweilzeit des Chlorgases in der Suspension. Durch die vollständige Umsetzung des zugesetzten Chlors beim Einleiten in die Reaktionsmischung wird der Endpunkt der Umsetzung (Auftreten von freiem Chlor im Abgas) genau erfaßt und ein Verlust von Chlor vermieden. Eine Umweltbelastung durch das Abgas (Chlorwasserstoff) aus der Umsetzung kann daher durch einfache Maßnahmen vermieden werden, beispielsweise durch Absorption des Abgases in Wasser, wobei wieder verwendbare Salzsäure entsteht. Die in Zhurnal Prikladnoi Khimii (s. o.) erzielte Ausbeute kann unter den dort beschriebenen Bedingungen nur bei sehr langen Reaktionszeiten (geringe Raum-Zeit-Ausbeute) und hohem Chlorüberschuß annähernd erreicht werden. Neben der Möglichkeit der diskontinuierlichen Reaktionsführung kann das erfindungsgemäße Verfahren auch kontinuierlich durchgeführt werden.

Zur Feststellung des Endes der Umsetzung kann der Chlorgehalt des Abgases herangezogen werden, weil der Chlorgehalt im Abgas beim Ende der Umsetzung stark ansteigt. Zur weiteren Erhöhung der Reaktionsgeschwindigkeit können zusätzlich Dispergiermittel, beispielsweise Natriumligninsulfonat, verwendet werden.

Die als Ausgangsprodukt verwendete Phenoxyessigsäure- bzw. 2-Methylphenoxyessigsäurelösung oder alpha-Phenoxypropionsäure bzw. alpha-2-Methylphenoxypropionsäure kann beispielsweise hergestellt werden durch die Umsetzung von Phenol oder 2-Methylphenol mit Chloressigsäure oder alpha-Chlorpropionsäure in alkalischem Medium. Besonders vorteilhaft werden hierbei Chloressigsäure oder alpha-Chlorpropionsäure, gegebenenfalls in wäßriger Lösung, und konzentrierte wäßrige Alkalihydroxidlösung, beispielsweise 50%ige Natronlauge, getrennt und gleichzeitig zu einer kochenden Lösung von Phenol oder 2-Methylphenol in einer organischen Flüssigkeit, die mit Wasser eine azeotrope Mischung bildet, beispielsweise Toluol oder Xylol, unter Auskreisen des vorhandenen bzw. durch die Reaktion gebildeten Wassers zugetropft. Beispielsweise wird nach der Umsetzung die organische Phase mit Wasser unterschichtet und nach der Trennung der Phasen und dem Ansäuern der wäßrigen Lösung die Chlorierung wie oben beschrieben kontinuierlich oder diskontinuierlich, drucklos oder unter Druck, mit oder ohne Reaktionsbeschleuniger durchgeführt. Nach dem Eingasen der erforderlichen Chlormenge wird die Mischung vorteilhaft noch 15 – 30 min bei der Reaktionstemperatur gerührt und danach abgesaugt.

## Beispiel 1

Zu einer kochenden Lösung von 94 Teilen (Gew.-Teile) Phenol in 500 Teilen Xylol tropft man getrennt und gleichzeitig innerhalb 60 Minuten eine Lösung von 98 Teilen Chloressigsäure in 50 Teilen Wasser und eine Lösung von 88 Teilen Natriumhydroxid in 90 Teilen Wasser. Man rührt 30 Minuten bei 140° C nach, gibt dann unter Rühren 500 Teile Wasser zu und trennt die wäßrige Phase ab. Dann fügt man unter kräftigem Rühren konz. Salzsäure bis pH 3 zu der wäßrigen Phase und leitet bei 50° C in 90 Minuten 150 Teile (Gew.-Teile) Chlorgas so ein, daß kein Chlor im Abgas auftritt. Man rührt 15 Minuten bei 50° C nach, saugt den Niederschlag ab und wäscht mit Wasser nach. Nach dem Trocknen erhält man 216 Teile (98% der Theorie, bezogen auf eingesetztes Phenol) einer 98%igen 2,4-Dichlorphenoxyessigsäure vom Schmelzpunkt 138 bis 140° C.

## Beispiel 2

Zu einer Mischung von 211,5 Teilen Phenol und 225 Teilen Wasser gibt man 267 Teile 50%ige (Gew.-%) Kalilauge, dann tropft man bei Rückfluß (105 – 108° C) in 60 Minuten 370 Teile einer 64%igen wäßrigen Lösung von Chloressigsäure zu, bis der pH-Wert der Lösung auf 10 abgesunken ist; dieser pH-Wert wird dann durch die gleichzeitige und getrennte Zugabe der Chloressigsäure und der Zugabe von 190 Teilen einer 50%igen wäßrigen Natronlauge eingehalten. Man rührt noch 60 Minuten bei Rückfluß, gibt dann 700 Teile Wasser zu und säuert unter kräftigem Rühren mit konz. Salzsäure bis pH 4 an. Man leitet bei 40 bis 50°C in 100 Minuten 173 Teile Chlorgas so ein, daß kein freies Chlor im Abgas auftritt. Man rührt noch 30 Minuten bei 50°C nach, saugt den Niederschlag ab, wäscht mit Wasser salzfrei und erhält nach dem Trocknen 487 Teile (98% der Theorie, bezogen auf eingesetztes Phenol) 97%ige 2,4-Dichlorphenoxyessigsäure vom Schmelzpunkt 137 bis 139°C.

### Beispiel 3

Zu einer Mischung aus 134,5 Teilen Phenol, 33 Teilen 50%iger (Gew.-%) wäßriger NaOH-Lösung, 46 Teilen 50%iger wäßriger KOH-Lösung und 50 Teilen Wasser tropft man innerhalb von zwei Stunden bei 105 bis 108°C getrennt und gleichzeitig eine Lösung von 94 Teilen Chloressigsäure in 60 Teilen Wasser und eine Mischung von 66 Teilen 50%iger wäßriger NaOH-Lösung und 93 Teilen 50%iger wäßriger KOH-Lösung. Man rührt noch 30 Minuten bei 105°C nach, setzt dann 900 Teile Wasser zu und extrahiert die Reaktionsmischung zweimal mit je 150 Teilen Methyl-tert.-butylether. In die wäßrige Phase leitet man bei 50°C in 5 Minuten 57 Teile Chlor ein, läßt die organische Phase absitzen, trennt die wäßrige Phase ab und setzt zur organischen Phase 1000 Teile Frischwasser von 60°C zu. Man vereinigt die wäßrigen Phasen und leitet bei 55°C in 15 Minuten 85 Teile Chlorgas in die wäßrige Phase ein, läßt 15 Minuten nachreagieren und saugt das Produkt bei 35°C ab. Ausbeute: 415 Teile (94% der Theorie) 2,4-Dichlorphenoxyessigsäure vom Schmelzpunkt 138 bis 141°C, Reinheit 97%.

### Beispiel 4

Zu einer kochenden Lösung von 338,4 Teilen Phenol in 2000 Teilen Toluol tropft man unter Auskreisen des Wassers in zwei Stunden getrennt und gleichzeitig 530 Teile 50%ige wäßrige NaOH-Lösung und 362 Teile alpha-Chlorpropionsäure (90%ig). Man rührt noch 1 Stunde bei 110°C nach, gibt dann 2000 Teile Wasser zu, trennt die organische Phase ab und extrahiert die wäßrige Phase bei pH 6 zweimal mit je 500 Teilen Toluol. Nach kurzem Durchleiten von Dampf leitet man in 35 Minuten bei 55°C 430 Teile Chlorgas in die wäßrige Phase ein und saugt dann das Reaktonsprodukt ab. Ausbeute: 652,2 Teile (92% der Theorie) alpha-2,4-Dichlorphenoxypropionsäure vom Schmelzpunkt 116 bis 118°C, Reinheit 98,5%.

### Beispiel 5

In eine Mischung aus 415 Teilen 2-Methylphenoxyessigsäure und 2500 Teilen Wasser leitet man in 50 Minuten bei 60°C 180 Teile Chlorgas ein. Nach dem Absaugen und Waschen mit Wasser erhält man 481,2 Teile (96% der Theorie) 2-Methyl-4-chlorphenoxyessigsäure vom Schmelzpunkt 116 bis 119°C, Reinheit 96%.

### Patentansprüche

1. Verfahren zur Herstellung von 2,4-Dichlor- bzw. 2-Methyl-4-chlorphenoxyessigsäure oder alpha-2,4-Dichlor- bzw. alpha-2-Methyl-4-chlorphenoxypropionsäure durch Umsetzung von Phenoxyessigsäure bzw. 2-Methylphenoxyessigsäure oder alpha-Phenoxypropionsäure bzw. alpha-2-Methylphenoxypropionsäure mit Chlor in Wasser, dadurch gekennzeichnet, daß man Phenoxyessigsäure bzw. 2-Methylphenoxyessigsäure oder alpha-Phenoxypropionsäure bzw. alpha-2-Methylphenoxypropionsäure aus ihrer wäßrigen Lösung ausfällt, in die so erhaltene Suspension der Säure in Wasser Chlorgas bei einer Temperatur von 30 bis 70°C höchstens in der Menge und Geschwindigkeit, in der es von der Suspension verbraucht wird, einleitet, bis 100 bis 110% der theoretisch erforderlichen Menge an Chlorgas von der Suspension aufgenommen worden sind, und die 2,4-Dichlor- bzw. 2-Methyl-4-chlorphenoxyessigsäure oder alpha-2,4-Dichlor- bzw. alpha-2-Methyl-4-chlor-phenoxypropionsäure von der Suspension abtrennt.

2. Verfahren nach Anspruch 1, dadurch gekennzeichnet, daß man als Ausgangsprodukt eine wäßrige Lösung von Phenoxyessigsäure bzw. 2-Methylphenoxyessigsäure oder alpha-Phenoxypropionsäure bzw. alpha-2-Methylphenoxypropionsäure verwendet, die durch Umsetzung von Phenol bzw. 2-Methylphenol bzw. ihren Alkalisalzen mit Chloressigsäure oder alpha-Chlorpropionsäure bzw. ihrem Alkalisalz in Wasser oder einem organischen Lösungsmittel und Extraktion der so erhaltenen Phenoxyessigsäure bzw. 2-Methylphenoxyessigsäure oder alpha-Phenoxypropionsäure bzw. alpha-2-Methylphenoxypropionsäure aus dem organischen Lösungsmittel mit Wasser erhalten wurde.

3. Verfahren nach Anspruch 1, dadurch gekennzeichnet, daß man eine Lösung von Phenoxyessigsäure bzw. 2-Methylphenoxyessigsäure oder alpha-Phenoxypropionsäure bzw. alpha-2-Methylphenoxypropionsäure in Wasser verwendet, die je Mol Phenoxyessigsäure bzw. 2-Methylphenoxyessigsäure oder alpha-Phenoxypropionsäure bzw. alpha-2-Methylphenoxypropionsäure 20 bis 40 Mol Wasser enthält.

### Claims

1. A process for the preparation of 2,4-dichloro- or 2-methyl-4-chlorophenoxyacetic acid or $\alpha$-2,4-dichloro- or $\alpha$-2-methyl-4-chlorophenoxypropionic acid by reacting phenoxyacetic or 2-methylphenoxyacetic acid or $\alpha$-phenoxypropionic or $\alpha$-2-methylphenoxypropionic acid with

chlorine in water, wherein phenoxyacetic or 2-methylphenoxyacetic acid or $\alpha$-phenoxypropionic or $\alpha$-2-methylphenoxypropionic acid is precipitated from an aqueous solution thereof, chlorine gas is passed into the resulting suspension of the acid in water at from 30 to 70° C at a rate not exceeding the rate at which it is consumed in the suspension, until from 100 to 110% of the theoretically required amount of chlorine gas has been taken up by the suspension, and the 2,4-dichloro- or 2-methyl-4-chlorophenoxyacetic acid or $\alpha$-2,4-dichloro- or $\alpha$-2-methyl-4-chlorophenoxypropionic acid is isolated from the suspension.

2. A process as claimed in claim 1, wherein an aqueous solution of phenoxyacetic or 2-methylphenoxyacetic acid or $\alpha$-phenoxypropionic or $\alpha$-2-methylphenoxypropionic acid, which has been obtained by reacting phenol or 2-methylphenol or an alkali metal salt thereof with chloroacetic acid or $\alpha$-chloropropionic acid or an alkali metal salt thereof in water or an organic solvent and extracting the resulting phenoxyacetic or 2-methylphenoxyacetic acid or a $\alpha$-phenoxypropionic or $\alpha$-2-methylphenoxypropionic acid from the organic solvent with water, is used as the starting material.

3. A process as claimed in claim 1, wherein a solution of phenoxyacetic or 2-methylphenoxyacetic acid or $\alpha$-phenoxypropionic or $\alpha$-2-methylphenoxypropionic acid in water is used, which solution contains 20 to 40 moles of water per mole of phenoxyacetic or 2-methylphenoxyactic acid or $\alpha$-phenoxypropionic or $\alpha$-2-methylphenoxypropionic acid.

### Revendications

1. Procédé de préparation de l'acide dichloro-2,4 ou méthyl-2 chloro-4 phénoxy-acétique ou de l'acide $\alpha$-dichloro-2,4 ou $\alpha$-méthyl-2 chloro-4 phénoxy-propionique par réaction de l'acide phénoxy-acétique ou méthyl-2 phénoxy-acétique ou de l'acide $\alpha$-phénoxy-propionique ou $\alpha$-méthyl-2 phénoxy-propionique dans l'eau avec le chlore, caractérisé en ce que l'on précipité l'acide phénoxy-acétique ou méthyl-2 phénoxy-acétique ou l'acide $\alpha$-phénoxy-propionique ou $\alpha$-méthyl-2 phénoxy-propionique de sa solution aqueuse et on introduit dans la suspension dans l'eau de l'acide obtenue, à une température comprise entre 30 et 70° C, du chlore gazeux en une quantité et à une vitesse correspondant au maximum à la proportion et à la vitesse auxquelles celui-ci peut être consommé par la suspension et ce jusqu'à la fixation par la suspension de 100 à 110% de la proportion théoriquement nécessaire en chlore gazeux, puis on sépare de la suspension l'acide dichloro-2,4 ou méthyl-2 chloro-4 phénoxy-acéti-que ou l'acide $\alpha$-dichloro-2,4 ou $\alpha$-méthyl-2 chloro-4 phénoxy-propionique formé.

2. Procédé suivant la revendication 1, caractérisé en ce que l'on emploie comme produit de départ une solution aqueuse d'acide phénoxy-acétique ou d'acide méthyl-2 phénoxy-acétique ou de l'acide $\alpha$-phénoxy-propionique ou $\alpha$-méthyl-2 phénoxy-propionique, obtenu par réaction de phénol ou de méthyl-2 phénol ou de leurs sels de métaux alcalins dans l'eau ou dans un solvant organique avec l'acide chlor-acétique ou l'acide $\alpha$-chloro-propionique ou un sel de métal alcalin de celui-ci et extraction de l'acide phénoxy-acétique ou méthyl-2 phénoxy-acétique ou de l'acide $\alpha$-phénoxy-propionique ou $\alpha$-méthyl-2 phénoxy-propionique formé du solvant organique à l'aide d'eau.

3. Procédé suivant la revendication 1, caractérisé en ce que l'on emploie une solution d'acide phénoxy-acétique ou d'acide méthyl-2 phénoxy-acétique ou d'acide $\alpha$-phénoxy-propionique ou d'acide $\alpha$-méthyl-2 phénoxy-propionique dans l'eau, contenant par mole d'acide phénoxy-acétique, d'acide méthyl-2 phénoxy-acétique, d'acide $\alpha$-phénoxy-propionique ou d'acide $\alpha$-méthyl-2 phénoxy-propionique entre 20 et 40 moles d'eau.